# EUROPÄISCHE PATENTSCHRIFT

(11) **EP 1 761 175 B1**
(45) Veröffentlichungstag und Bekanntmachung des Hinweises auf die Patenterteilung: **20.04.2011**
(21) Anmeldenummer: 04740170.8
(22) Anmeldetag: 22.06.2004
(51) Int. Cl.: A61B 17/02, A61B 17/88

(54) **CHIRURGISCHER HEBEL**
SURGICAL LEVER
ECARTEUR CHIRURGICAL

(43) Veröffentlichungstag der Anmeldung: 14.03.2007
(73) Patentinhaber: WALDEMAR LINK GmbH & Co. KG, 22339 Hamburg (DE)
(72) Erfinder: LUBINUS, Philip, 24106 Kiel (DE); KELLER, Arnold, 23863 Kayhude (DE)
(74) Vertreter: Glawe, Delfs, Moll
(86) Internationale Anmeldenummer: PCT/EP2004/006742
(87) Internationale Veröffentlichungsnummer: WO 2005/122910

(56) Entgegenhaltungen:
- US-A- 4 995 875
- ANONYM: "APC Proximal Femoral Elevator" [Online] 2000, INNOMED , SAVANNAH , VS , XP002317990 Produktliteratur Gefunden im Internet: URL:http://www.innomed.net/PDFsInnomed/Inn oAPCProxFemElevator.pdf> [gefunden am 2005-02-16] Urheberrechtsdatum 2000
- ANONYM: "Amstutz Femoral Neck Elevator" [Online] 2003, INNOMED , SAVANNAH, VS , XP002317991 Produktliteratur Gefunden im Internet: URL:http://www.innomed.net/PDFsInnomed/Inn oAmstutzRetractors.pdf> [gefunden am 2005-02-16] Urheberrechtsdatum 2003

## Beschreibung

Die Erfindung betrifft einen chirurgischen Hebel, insbesondere zum Abheben des Femurs vom Beckenknochen.

Derartige chirurgische Hebel werden typischerweise bei Operationen verwendet, mit denen das natürliche Hüftgelenk durch ein künstliches ersetzt wird, um nach der Resektion des Femurkopfes das Femur so zu halten, dass der femorale Markraum aufgearbeitet werden kann. Falls die Resektion im ausgerenkten Zustand des Hüftgelenks vorgenommen wird, besteht zusätzlich auch die Möglichkeit, das Femur während der Resektion vom Beckenknochen abzuheben.

Bei den bekannten chirurgischen Hebeln, mit denen das Femur vom Beckenknochen abgehoben wird, bildet der Rand des Acetabulums das Widerlager, auf dem der chirurgische Hebel abgestützt wird. Dies ist nachteilhaft, weil zum einen Strukturen am Rand des Acetabulums beschädigt werden können und zum anderen der Rand des Acetabulums kein sicheres Widerlager bietet, das beispielsweise ein seitliches Abrutschen verhindert. Ferner ist es bekannt (US 4995875), den Hebel zwischen seinem Betätigungsgriff und seinem zum Angriff am Femur vorgesehenen Teil mit einem Vorsprung zu versehen, der zur Abstützung in der Tiefe des Acetabulums bemessen ist und eine konvexe Abstützfläche aufweist. Da sich die Abstützfläche im Acetabulum zentriert, kann der Hebel nicht abrutschen. Da die Abstützfläche konvex ist und insoweit der Form des Acetabulums mehr oder weniger angepaßt ist, ist auch eine Beschädigung weniger zu befürchten. Jedoch hat der bekannte Hebel den Nachteil, daß die konvexe Abstützfläche durch eine Schraubverbindung mit dem eigentlichen Hebel verbunden ist, die schwerlich einen Austausch der Abstützfläche während der Operation gestattet.

Der Erfindung liegt die Aufgabe zu Grunde, einen chirurgischen Hebel der letztgenannten Art zur Verfügung zu stellen, bei dem die Abstützfläche während der Operation leicht ausgetauscht werden kann und dennoch einen festen Sitz am Hebel hat. Die erfindungsgemäße Lösung besteht in den Merkmalen des Anspruchs 1.

Zwischen dem Betätigungsgriff und der Angriffseinrichtung ist ein zur Abstützung in der Tiefe des Acetabulums bemessener Vorsprung angeordnet ist. Die Tiefe des Acetabulums bietet wegen ihrer konkaven Form ein stabiles Widerlager für den chirurgischen Hebel. Dank der Konkavität des Acetabulums findet der in Position gebrachte Vorsprung darin sicheren Halt. Die Abstützung in der Tiefe des Acetabulums gibt Sicherheit bei jeglicher Kraftrichtung. Das Femur kann in unterschiedliche Richtungen vom Beckenknochen abgehoben werden.

Der Vorsprung des chirurgischen Hebels weist eine Abstützfläche auf, die entsprechend der Krümmung des Acetabulums konvex geformt ist. Durch diese Formung werden lokale Belastungen des Acetabulums vermieden, da die Abstützfläche auf eine größere Fläche wirkt.

Es sind Abstützflächen mit unterschiedlichen Krümmungsradien verfügbar, die alternativ je nach Größe oder Krümmungsradius des Acetabulums zum Einsatz kommen. Die Acetabula verschiedener Menschen sind unterschiedlich geformt und haben insbesondere verschiedene Durchmesser. Durch die Abstützflächen mit unterschiedlichen Krümmungsradien wird der Einsatz des chirurgischen Hebels bei einer möglichst großen Vielzahl von Menschen ermöglicht. Es sind mehrere auswechselbare, unterschiedlich gekrümmte Abstützflächen bildende Teile des Vorsprungs vorhanden.

In einer vorteilhaften Ausführungsform hat die Abstützfläche eine mindestens halbsphärische Form. Dadurch ist es besonders einfach, sie in das Acetabulum einzuführen, und sie zentriert sich darin selbst.

Die Verbindung zwischen chirurgischem Hebel und Abstützfläche wird über eine Konus-Steckverbindung hergestellt, wobei der Konuswinkel so gewählt ist, dass die Abstützfläche sich einerseits nicht unbeabsichtigt löst und andererseits gegen eine andere durch einfaches Abziehen und Aufstecken ausgetauscht werden kann. Der die Abstützfläche bildende Teil kann starr mit dem Hebel verbunden sein. Bei der Hebelbewegung gleitet dann die Abstützfläche im Acetabulum. konkav angepasste Form ist geeignet, ein Abrutschen des Trochanters von dem chirurgischen Hebel zu verhindern.

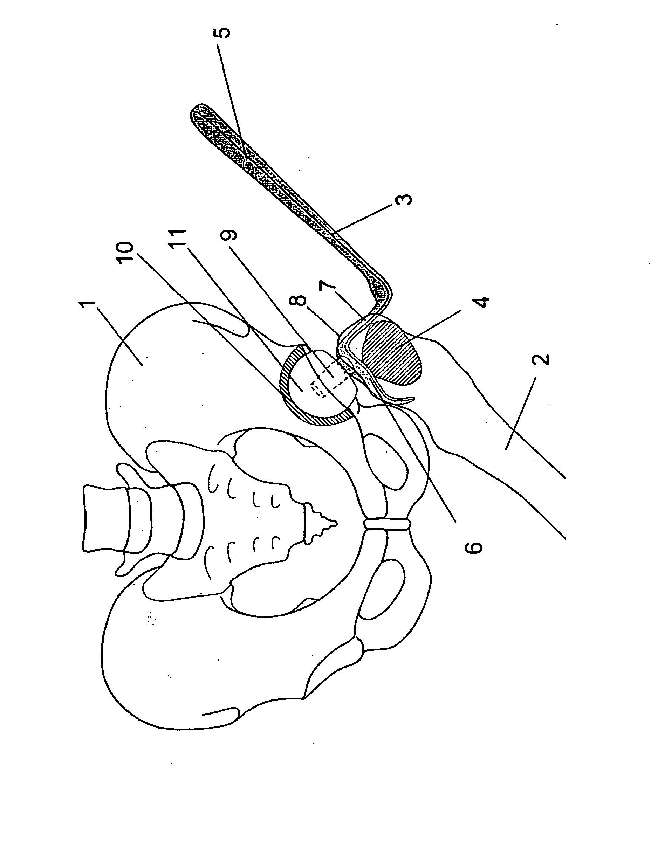

Die Erfindung wird im Folgenden unter Bezugnahme auf die beiliegende Zeichnung anhand eines vorteilhaften Ausführungsbeispiels beschrieben. Die einzige Figur zeigt eine Draufsicht auf einen erfindungsgemäßen chirurgischen Hebel in der Anwendung an einem Hüftgelenk.

Dargestellt sind ein menschlicher Beckenknochen und ein Teil des linken Femurs 2, dessen Femurkopf im Bereich des Femurhalses reseziert und entfernt ist. Die Resektionsfläche 4 und das Acetabulum 10 liegen frei. Das Femur 2 ist mit Hilfe eines erfindungsgemäßen chirurgischen Hebels 3 vom Beckenknochen 1 abgehoben und derart nach innen rotiert, dass der femorale Markraum zur Bearbeitung durch den Operateur zugänglich ist. Der chirurgische Hebel 3 greift am großen Trochanter 8 des Femurs 2 an.

Der chirurgische Hebel 3 umfasst einen Betätigungsgriff 5 und eine Angriffseinrichtung 6. Der Betätigungsgriff 5 und die Angriffseinrichtung 6 sind längliche Elemente, die an ihren inneren Enden über ein im wesentlichen senkrecht zu beiden stehendes Zwischenstück 7 starr verbunden sind. Die Angriffseinrichtung 6 ist an ihrem Ende durch Bildung einer zurückgebogenen Nase konkav geformt und damit an die Außenform des großen Trochanters 8 angepasst.

Am inneren Ende der Angriffseinrichtung 6 ist ein Konus 9 angeordnet. Der Konus 9 bildet - etwas in Richtung des äußeren Endes der Angriffseinrichtung 6 verschoben - eine Verlängerung des Zwischenstücks 7. Mit dem Konus 9 über eine Konus-Steckverbindung verbunden ist ein Kugelsegment 11, in dessen Schnittfläche sich eine zu dem Konus 9 komplementäre konusförmige Bohrung befindet. Die Außenfläche des Kugelsegments 11 befindet sich im Acetabulum 10 und ist als Abstützfläche entsprechend der Krümmung des Acetabulums 10 geformt. Der Konus 9 bildet zusammen mit dem Kugelsegment 11 einen Vorsprung, mit dem der chirurgische Hebel 3 in der Tiefe des Acetabulums 10 abgestützt wird.

Das Kugelsegment 11 wird vom Acetabulum 10 sicher gehalten. Auch durch Kräfte, die quer zum Konus 9 wirken, wird das Kugelsegment 11 nicht aus seiner Position gebracht. Weiterhin kann das Kugelsegment 11 innerhalb des Acetabulums 10 in alle Richtungen gedreht werden, so dass der chirurgische Hebel sowohl aus unterschiedlichen Richtungen als auch in unterschiedlichen Winkeln angesetzt werden kann. Über das Kugelsegment 11 bildet das Acetabulum 10 ein Widerlager, das eine sichere und vielseitige Verwendung des chirurgischen Hebels 3 möglich macht.

## Patentansprüche

1. Chirurgischer Hebel, insbesondere zum Abheben des Femurs (2) vom Beckenknochen (1), mit einem Betätigungsgriff (5), einer Angriffseinrichtung (6) und einem zwischen dem Betätigungsgriff (5) und der Angriffseinrichtung (6) angeordneten, zur Abstützung in der Tiefe des Acetabulums bemessenen Vorsprung (9, 11), der einen auswechselbaren, eine konvexe Abstützfläche bildenden Teil (11) aufweist, **dadurch gekennzeichnet, dass** der die Abstützfläche bildende Teil (11) über eine Konus-Steckverbindung mit dem chirurgischen Hebel verbunden ist.

2. Chirurgischer Hebel nach Anspruch 1, **dadurch gekennzeichnet, dass** die Abstützfläche mindestens halbsphärisch geformt ist.

3. Chirurgischer Hebel nach Anspruch 1 oder 2, **dadurch gekennzeichnet, dass** die Angriffseinrichtung (6) in Wirkrichtung konkav geformt ist.

## Claims

1. Surgical lever, particularly for lifting the femur (2) from the hip bone (1), with an actuating handle (5), an engagement device (6), and a projection (9, 11) dimensioned for support within the depth of the acetabulum and arranged between the actuating handle (5) and the engagement device (6), which projection has an exchangeable part (11) forming a convex support surface, **characterized in that** the part (11) forming the support surface is connected to the surgical lever via a conical plug connection.

2. Surgical lever according to Claim 1, **characterized in that** the support surface has an at least hemispherical configuration.

3. Surgical lever according to Claim 1 or 2, **characterized in that** the engagement device (6) has a concave configuration in the working direction.

## Revendications

1. Ecarteur chirurgical, en particulier pour écarter le fémur (2) de l'os du bassin (1), comprenant un manche de commande (5), un dispositif d'intervention (6) et une saillie (9, 11) disposée entre le manche de commande (5) et le dispositif d'intervention (6), dimensionnée pour s'appuyer dans la profondeur de l'acétabule, qui présente une partie (11) interchangeable, formant une surface d'appui convexe, **caractérisé en ce que** la partie (11) formant la surface d'appui est connectée par le biais d'une liaison par enfichage conique à l'écarteur chirurgical.

2. Ecarteur chirurgical selon la revendication 1, **caractérisé en ce que** la surface d'appui est formée au moins sous forme hémisphérique.

3. Ecarteur chirurgical selon la revendication 1 ou 2, **caractérisé en ce que** le dispositif d'intervention (6) est formé sous forme concave dans la direction d'action.
